# EUROPÄISCHE PATENTSCHRIFT

(11) **EP 3 202 307 B1**
(45) Veröffentlichungstag und Bekanntmachung des Hinweises auf die Patenterteilung: **24.10.2018**
(21) Anmeldenummer: 17000129.1
(22) Anmeldetag: 26.01.2017
(51) Int. Cl.: A61B 1/267, A61B 1/05, A61B 1/012

(54) **LARYNGOSKOP**
LARYNGOSCOPE
LARYNGOSCOPE

(30) Priorität: 05.02.2016 DE 102016001309
(43) Veröffentlichungstag der Anmeldung: 09.08.2017
(73) Patentinhaber: Karl Storz SE & Co. KG, 78532 Tuttlingen (DE)
(72) Erfinder: Merz, Ulrich, 78532 Tuttlingen (DE); Attinger, Juerg, 8260 Stein am Rhein (CH); Fuhr, Eugenia, 78532 Tuttlingen (DE); Breinlinger, Thomas, 78532 Tuttlingen (DE)
(74) Vertreter: Hofmeister, Frank

(56) Entgegenhaltungen:
- WO-A2-2016/074894
- US-A1- 2007 106 121
- US-A1- 2010 261 967
- US-A1- 2013 060 089
- US-A1- 2013 104 884

## Beschreibung

Die Erfindung betrifft ein Laryngoskop mit einem Handgriff, an dessen distalem Ende in einem Winkel zur Längsachse des Handgriffs ein Spatel angeordnet ist, wobei im Handgriff und im Spatel ein Kanal zur Aufnahme eines Bildleiters eines Video-Endoskops so ausgebildet ist, dass der Kanal in einem Übergangsbereich vom Handgriff zum Spatel in einem Radius vom Handgriff in den Spatel übergeht, wobei im als Radius ausgebildeten Übergangsbereich vom Handgriff in den Spatel im Inneren des Kanals mindestens eine als Führung für den Bildleiter dienende Anlaufschräge ausgebildet ist.

Laryngoskope dienen zur direkten Betrachtung und Untersuchung des Kehlkopfes. Ein Laryngoskop ist beispielsweise aus der DE 199 55 180 B4 bekannt. Bei dem bekannten, als Video-Laryngoskop ausgebildeten Laryngoskop dient der Bildleiter dazu, ein Bild im Bereich des distalen Endes des Spatels aufzunehmen und an eine vom Spatel räumlich getrennte Bildwiedergabeeinheit zu übertragen. Der Bildleiter besteht bei den Video-Laryngoskopen in der Regel aus einem Kabel, an dessen distalem Ende ein Video-Chip angeordnet ist.

Da der Spatel im Wesentlichen rechtwinklig zur Längsachse des Handgriffs am distalen Ende des Handgriffs angeordnet ist, weist auch der im Handgriff und im Spatel ausgebildete Kanal zur Aufnahme des Bildleiters eines Video-Endoskops im Übergangsbereich vom Handgriff zum Spatel eine starke Abwinklung auf, die in der Praxis als Radius ausgebildet ist.

Ein weiteres Video-Laryngoskop ist beispielsweise das C-MAC® S der Karl Storz GmbH & Co. KG.

Beim Einführen des Bildleiters eines Video-Endoskops in den Kanal besteht die Gefahr, dass der Bildleiter in dem abgewinkelten Übergangsbereich vom Handgriff in den Spatel gestaucht wird, was zu einer bleibenden Schädigung des Bildleiters und somit einer Verschlechterung der Bildübertragung führen kann.

Ein gattungsgemäßes Laryngoskop ist beispielsweise aus der US 2010/0261967 A1 bekannt. Diese bekannte Laryngoskop weist einen Handgriff auf, an dessen distalem Ende ein gegenüber der Längsachse des Handgriffs abgewinkelter Spatel angeordnet ist. Im Handgriff und im Spatel ist ein Kanal für den Bildleiter eines Video-Endoskops so ausgebildet, dass der Kanal im Übergangsbereich vom Handgriff in den Spatel in einem Radius in den Spatel übergeht, wobei im Übergangsbereich vom Handgriff zum Spatel eine umlaufende Schräge ausgebildet.

Diese den Eintritt in den Spatel bildende Schräge stellt einen scharfkantigen Übergang in den Radius des im Spatel ausgebildeten Kanals dar, so dass auch bei dieser bekannten Ausführungsform die Gefahr einer Stauchung des Bildleiters besteht.

Ein weiteres Laryngoskop ist aus der WO 2016/074894 A2 bekannt. Dieses bekannte Laryngoskop weist einem Handgriff an dessen distalem Ende ein gegenüber der Längsachse des Handgriffs abgewinkelter Spatel angeordnet ist. Im Handgriff und im Spatel ist ein Kanal ür den Bildleiter eines Video-Endoskops so ausgebildet, dass der Kanal im Übergangsbereich vom Handgriff in den Spatel in einem Radius in den Spatel übergeht.

Der Eintritt in den Kanal ist bei diesem Laryngoskop als trichterförmige Öffnung ausgebildet. Diese trichterförmige Öffnung stellt einen abrupten Übergang in den Radius des Kanals darstellt, so dass auch bei dieser bekannten Ausführungsform die Gefahr einer Stauchung des Bildleiters besteht.

Davon ausgehend liegt der Erfindung die **Aufgabe** zugrunde, ein Laryngoskop zu schaffen, das bei einfacher Handhabung ein materialschonendes Einführen des Bildleiters in den Bildleiterkanal gewährleistet.

Die **Lösung** dieser Aufgabenstellung ist erfindungsgemäß durch die Merkmale von Anspruch 1 definiert, und dadurch gekennzeichnet, dass die mindestens eine Anlaufschräge aus mehreren mit Abstand zueinander angeordneten Rippen besteht, wobei die Rippen in einem Winkel zur Längsachse des Handgriffs in den Radius hinein weisend an der Kanalinnenwand angeordnet sind.

Durch die Ausbildung einer als Führung für den Bildleiter dienenden Anlaufschräge im Inneren des Kanals ist es möglich, den Bildleiter stauchungsfrei auch um enge Radien zu führen.

Dadurch, dass die Anlaufschräge aus mehreren mit Abstand zueinander angeordneten Rippen besteht, verringert sich das Gewicht, da weniger Material für die Anlaufschräge verwendet werden muss und bietet darüber hinaus durch die parallel zueinander verlaufenden Rippen eine sehr gute Führung für den in den Kanal einzuführenden Bildleiter.

Die winklige Ausrichtung der Rippen ist dabei so ausgeführt, dass die Rippen in den Radius hinein weisend an der Kanalinnenwand angeordnet sind.

Vorteilhafterweise sind die mit Abstand zueinander angeordneten Rippen parallel zueinander ausgerichtet.

Mit einer bevorzugten Ausführungsform zur Ausbildung der gerippten Anlaufschräge wird vorgeschlagen, dass die Rippen, in Einsteckrichtung des Bildleiters betrachtet, proximalseitig der stärksten Krümmung des Radius an der Kanalinnenwand ausgebildet sind. Diese Anordnung der Rippen in Einschubrichtung vor der stärksten Abwinklung stellt sicher, dass der Bildleiter im richtigen Winkel und ohne Stauchgefahr in den Übergangsbereich zwischen Handgriff und Spatel geleitet wird.

Das lagegerechte Einführen des Bildleiters in den Kanal kann erfindungsgemäß weiterhin dadurch erleichtert werden, dass die Rippen auch noch den lichten Durchmesser des Kanals verengen, da sie über ihre axiale Länge von proximal nach distal eine ansteigende radiale Dicke aufweisen.

Weiterhin wird mit der Erfindung vorgeschlagen, dass die Rippen vollumfänglich über den gesamten Umfang der Kanalinnenwand ausgebildet sind.

Gemäß einer alternativen Ausführungsform der Erfindung wird vorgeschlagen, dass die Rippen nur abschnittsweise über den Umfang der Kanalinnenwand ausgebildet sind. Bei der nur abschnittsweisen Ausbildung der Rippen können diese auf einen oder mehrere Abschnitte über den Umfang der Kanalinnenwand aufgeteilt sein.

Der Winkel, unter dem der Spatel gegenüber der Längsachse des Handgriffs abgewinkelt ist, beträgt vorzugsweise 70° bis 90°.

Schließlich wird mit der Erfindung vorgeschlagen, dass der Handgriff und der Spatel lösbar miteinander verbunden sind. Diese Ausgestaltungsform ermöglicht die Verwendung verschieden geformter Spatel bei Beibehaltung des Handgriffs.

Auch wenn Laryngoskope in der Praxis aus einem Kunststoffmaterial gefertigt einstückig oder aus zwei Halbschalen bestehend ausgebildet sind und häufig als Einwegartikel verwendet werden, kann diese zweiteilige Ausgestaltungsform sinnvoll sein, um eine möglichst große Bandbreite an Laryngoskopformen vorrätig zu halten.

Weitere Merkmale und Vorteile der Erfindung ergeben sich anhand der zugehörigen Zeichnungen, in denen ein Ausführungsbeispiel eines erfindungsgemäßen Laryngoskops nur beispielhaft dargestellt ist, ohne die Erfindung auf dieses Ausführungsbeispiel zu beschränken. In den Zeichnungen zeigt:
- Fig. 1: eine Seitenansicht eines erfindungsgemäßen Laryngoskops mit zugehörigem Video-Endoskop vor dem Zusammenfügen;
- Fig.2: eine Seitenansicht gemäß Fig. 1, jedoch das Laryngoskop mit eingesetztem Video-Endoskop darstellend;
- Fig. 3: eine um 180° gedrehte geschnittene Seitenansicht des Laryngoskops gemäß Fig. 1 und
- Fig. 4: eine schematische Seitenansicht einer Rippe.

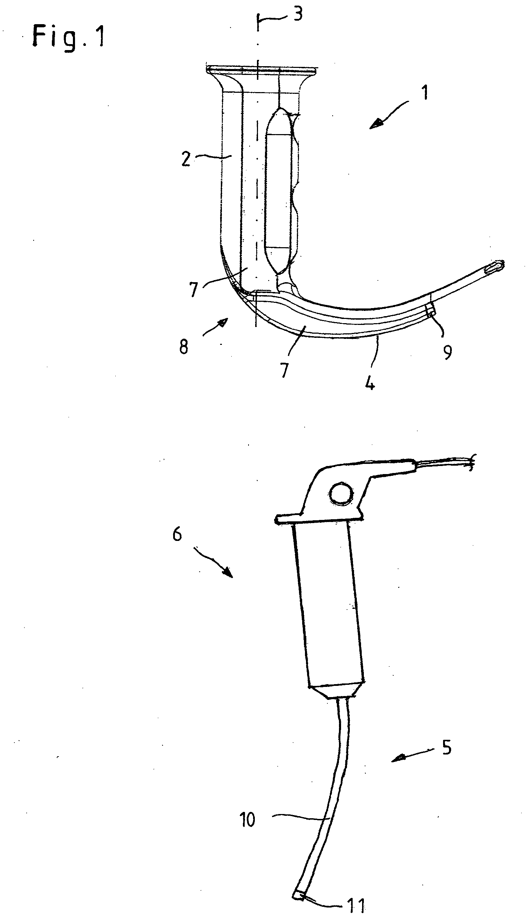

Das in den Abbildungen Fig. 1 bis 3 dargestellte Laryngoskop 1 besteht aus einem Handgriff 2 sowie einem am distalen Ende des Handgriffs 2 unter einem Winkel zur Längsachse 3 des Handgriffs 2 angeordneten Spatel 4.

Wie aus den Abbildungen ersichtlich, ist der Spatel 4 im Wesentlichen rechtwinklig zur Längsachse 3 des Handgriffs 2 ausgerichtet. In der Praxis beträgt der Winkel, unter dem der Spatel 4 gegenüber der Längsachse 3 des Handgriffs 2 abgewinkelt ist, 70° bis 90°.

Derartige als Einwegartikel verwendete Laryngoskope 1 bestehen in der Regel aus einem Kunststoffmaterial und werden vorzugsweise aus zwei im Spritzgussverfahren hergestellten Halbschalen gefertigt.

Zum Einführen eines Bildleiters 5 eines Video-Endoskops 6 ist im Laryngoskop 1 ein Kanal 7 ausgebildet, der im Übergangsbereich 8 vom Handgriff 2 zum Spatel 4 in einem Radius vom Handgriff 2 in den Spatel 4 übergeht. Der vom Handgriff 2 bis in den Spatel 4 reichende Kanal 7 ist zum Einführen des Bildleiters 5 proximalseitig im Handgriff 2 offen ausgebildet, während das im Spatel gelegene distale Ende des Kanals 7 in der Regel durch ein Fenster 9 verschlossen ist.

Bei dem hier als Bildleiter 5 des Laryngoskops 1 bezeichneten Bauteil handelt es sich um ein Kabel 10, an dessen distalem Ende ein Video-Chip 11 angeordnet ist.

Da einerseits der Spatel 4 in einem Winkel von 70° bis 90° gegenüber der Längsachse 3 des Handgriffs 2 abgewinkelt ist und somit auch der Radius in dem der Kanal 7 im Übergangsbereich 8 vom Handgriff 2 in den Spatel 4 sehr eng ist und andererseits der Bildleiter 5 des Video-Endoskops 6 eine gewisse Steifigkeit aufweist, besteht beim Einführen des Bildleiters 5 in den Kanal 7 die Gefahr, dass der Bildleiter 5 im Radius des Übergangsbereichs 8 aufsetzt und gestaucht wird. Diese Stauchungen des Bildleiters 5 können zu einer bleibenden Schädigung des Bildleiters 5 und somit einer Verschlechterung der Bildübertragung führen.

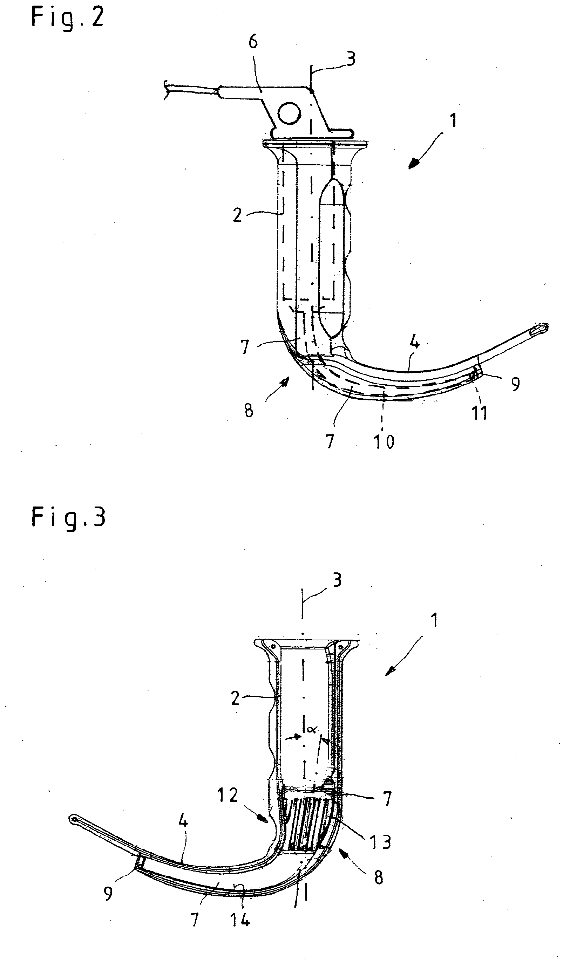

Um ein stauchungsfreies Einführen des Bildleiters 5 durch den Kanal 7 vom Handgriff 2 hinein in den Spatel 4 zu ermöglichen, ist bei dem in den Abbildungen Fig. 1 und 2 dargestellten Laryngoskop 1, wie insbesondere aus Fig. 3 ersichtlich, im Übergangsbereich 8 vom Handgriff 2 in den Spatel 4 im Inneren des Kanals 7 mindestens eine Anlaufschräge 12 ausgebildet. Die den Kanal 7 verjüngende Anlaufschräge 12 dient im Inneren des Kanals 7 als Führung für den Bildleiter 5 und ermöglicht so, den Bildleiter 5 stauchungsfrei auch um enge Radien zu führen.

Wie aus Fig. 3 ersichtlich, besteht die Anlaufschräge 12 aus mehreren parallel und mit Abstand zueinander angeordneten Rippen 13. Diese Ausgestaltung verringert das Gewicht, da weniger Material für die Anlaufschräge 12 verwendet werden muss und bietet darüber hinaus durch die parallel zueinander verlaufenden Rippen 13 eine sehr gute Führung für den in den Kanal 7 einzuführenden Bildleiter 5.

Um das zielgerichtete Einführen des Bildleiters 5 in den Kanal 7 zu erleichtern, sind die Rippen 13 in den Kanal 7 weisend in einem Winkel α zur Längsachse 3 des Handgriffs 2 ausgerichtet. Diese winklige Ausrichtung der Rippen 13 ist dabei so ausgeführt, dass die Rippen 13 in den Radius hinein weisend an der Kanalinnenwand 14 des Kanals 7 angeordnet sind.

Die die Anlaufschräge 12 bildenden Rippen 13 sind, in Einsteckrichtung des Bildleiters 5 in den Kanal 7 betrachtet, proximalseitig der stärksten Krümmung des Radius an der Kanalinnenwand 14 ausgebildet. Diese Anordnung der Rippen 13 in Einschubrichtung vor der stärksten Abwinklung stellt sicher, dass der Bildleiter 5 im richtigen Winkel und ohne Stauchgefahr in den Übergangsbereich 8 zwischen Handgriff 2 und Spatel 4 geleitet wird.

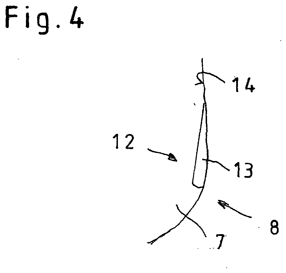

Wie aus Fig. 4 ersichtlich, können die Rippen 13 zusätzlich so ausgebildet sein, dass sie zusätzlich zur Abwinklung um den Winkel α zur Längsachse 3 des Handgriffs 2 auch noch den lichten Durchmesser des Kanals 7 verengen, da sie über ihre axiale Länge von proximal nach distal eine ansteigende radiale Dicke aufweisen.

Die Anordnung der Rippen 13 an der Kanalinnenwand 14 kann so ausgeführt sein dass die Rippen 13 entweder vollumfänglich über den gesamten Umfang der Kanalinnenwand 14 ausgebildet sind oder aber nur abschnittsweise über den Umfang der Kanalinnenwand 14 ausgebildet sind. Bei der abschnittsweisen Ausbildung der Rippen 13 können diese auf einen oder mehrere Abschnitte über den Umfang der Kanalinnenwand 14 aufgeteilt sein.

Ein wie zuvor beschrieben ausgebildetes Laryngoskop 1 gewährleistet bei einfacher Handhabung ein materialschonendes Einführen des Bildleiters 5 in den Kanal 7.

## Patentansprüche

1. Laryngoskop mit einem Handgriff (2), an dessen distalem Ende in einem Winkel zur Längsachse (3) des Handgriffs (2) ein Spatel (4) angeordnet ist, wobei im Handgriff (2) und im Spatel (4) ein Kanal (7) zur Aufnahme eines Bildleiters (5) eines Video-Endoskops (6) so ausgebildet ist, dass der Kanal (7) in einem Übergangsbereich (8) vom Handgriff (2) zum Spatel (4) in einem Radius vom Handgriff (2) in den Spatel (4) übergeht, wobei im als Radius ausgebildeten Übergangsbereich (8) vom Handgriff (2) in den Spatel (4) im Inneren des Kanals (7) mindestens eine als Führung für den Bildleiter (5) dienende Anlaufschräge (12) ausgebildet ist,
**dadurch gekennzeichnet,**
**dass** die mindestens eine Anlaufschräge (12) aus mehreren mit Abstand zueinander angeordneten Rippen (13) besteht, wobei die Rippen (13) in einem Winkel (α) zur Längsachse (3) des Handgriffs (2) in den Radius hinein weisend an der Kanalinnenwand (14) angeordnet sind.

2. Laryngoskop nach Anspruch 1, **dadurch gekennzeichnet, dass** die Rippen (13) parallel zueinander angeordnet sind.

3. Laryngoskop nach Anspruch 1 oder 2, **dadurch gekennzeichnet, dass** die Rippen (13), in Einsteckrichtung des Bildleiters (5) betrachtet, proximalseitig der stärksten Krümmung des Radius an der Kanalinnenwand (14) des Kanals (7) ausgebildet sind.

4. Laryngoskop nach einem der Ansprüche 1 bis 3, **dadurch gekennzeichnet, dass** die Rippen (13) über ihre axiale Länge von proximal nach distal eine ansteigende radiale Dicke aufweisen.

5. Laryngoskop nach einem der Ansprüche 1 bis 4, **dadurch gekennzeichnet, dass** die Rippen (13) vollumfänglich über den gesamten Umfang der Kanalinnenwand (14) ausgebildet sind.

6. Laryngoskop nach einem der Ansprüche 1 bis 4, **dadurch gekennzeichnet, dass** die Rippen (13) nur abschnittsweise über den Umfang der Kanalinnenwand (14) ausgebildet sind.

7. Laryngoskop nach einem der Ansprüche 1 bis 6, **dadurch gekennzeichnet, dass** der Winkel, unter dem der Spatel (4) gegenüber der Längsachse (3) des Handgriffs (2) abgewinkelt ist, 70° bis 90° beträgt.

8. Laryngoskop nach einem der Ansprüche 1 bis 7, **dadurch gekennzeichnet, dass** der Handgriff (2) und der Spatel (4) lösbar miteinander verbunden sind.

## Claims

1. A laryngoscope with a handle (2), at the distal end of which a spatula (4) is arranged at an angle to the longitudinal axis (3) of the handle (2), wherein a channel (7) for accommodating an image guide (5) of a video endoscope (6) is formed in the handle (2) and in the spatula (4) such that the channel (7) merges in a radius from the handle (2) into the spatula (4) in a transition area (8) from the handle (2) to the spatula (4), wherein, in the transition area (8) from the handle (2) into the spatula (4), at least one angled ramp (12) is formed in the interior of the channel (7) as a guide for the image carrier (5),
**characterized in that**
the at least one angled ramp (12) is composed of several ribs (13) arranged at a distance from one another, wherein the ribs (13), facing toward the channel inner wall (14), are arranged at an angle (a) to the longitudinal axis (3) of the handle (2) in the radius.

2. The laryngoscope according to claim 1, **characterized in that** the ribs (13) are arranged parallel to one another.

3. The laryngoscope according to either claim 1 or 2, **characterized in that** the ribs (13), viewed in the direction of insertion of the image carrier (5), are formed on the inner wall (14) of the channel (7) at a location proximal from the strongest curvature of the radius.

4. The laryngoscope according to any of claims 1 to 3, **characterized in that** the ribs (13) have an increasing radial thickness along their axial length, from proximal to distal.

5. The laryngoscope according to any of claims 1 to 4, **characterized in that** the ribs (13) are formed all the way round the entire circumference of the inner wall (14) of the channel.

6. The laryngoscope according to any of claims 1 to 4, **characterized in that** the ribs (13) are formed only in sections around the circumference of the inner wall (14) of the channel.

7. The laryngoscope according to any of claims 1 to 6, **characterized in that** the angle at which the spatula (4) is tilted with respect to the longitudinal axis (3) of the handle (2) is from 70° to 90°.

8. The laryngoscope according to any of claims 1 to 7, **characterized in that** the handle (2) and the spatula (4) are connected to each other in a detachable manner.

## Revendications

1. Laryngoscope avec un manche (2), à l'extrémité distale duquel est disposée une lame (4) sous un angle avec l'axe longitudinal (3) du manche (2), où dans le manche (2) et dans la lame (4) est formé un canal (7) pour recevoir un conducteur d'image (5) d'un endoscope vidéo (6) de telle manière que le canal (7) dans un domaine de transition (8) du manche (2) à la lame (4) passe dans un rayon du manche (2) dans la lame (4), où dans le domaine de transition (8) du manche (2) à la lame (4) formé comme rayon au moins une partie oblique initiale (12) servant de guidage pour le conducteur d'image (5) est formée à l'intérieur du canal (7),
**caractérisé en ce que** la au moins une partie oblique initiale (12) consiste en plusieurs nervures (13) disposées à distance les unes des autres, où les nervures (13) sont disposées au niveau de la paroi interne de canal (14) sous un angle (a) avec l'axe longitudinal (3) du manche (2) en étant tournées vers le rayon.

2. Laryngoscope selon la revendication 1, **caractérisé en ce que** les nervures (13) sont disposées parallèlement les unes aux autres.

3. Laryngoscope selon la revendication 1 ou 2, **caractérisé en ce que** les nervures (13), observées dans la direction d'insertion du conducteur d'image (5), sont formées du côté proximal de la plus forte courbure du rayon au niveau de la paroi interne de canal (14) du canal (7).

4. Laryngoscope selon l'une des revendications 1 à 3, **caractérisé en ce que** les nervures (13) présentent une épaisseur radiale croissante de proximal à distal sur leur longueur axiale.

5. Laryngoscope selon l'une des revendications 1 à 4, **caractérisé en ce que** les nervures (13) sont formées sur toute la périphérie sur toute la périphérie de la paroi interne de canal (14).

6. Laryngoscope selon l'une des revendications 1 à 4, **caractérisé en ce que** les nervures (13) ne sont formées que par segments sur la périphérie de la paroi interne de canal (14).

7. Laryngoscope selon l'une des revendications 1 à 6, **caractérisé en ce que** l'angle sous lequel la lame (4) est coudée par rapport à l'axe longitudinal (3) du manche (2) est de 70° à 90°.

8. Laryngoscope selon l'une des revendications 1 à 7, **caractérisé en ce que** le manche (2) et la lame (4) sont reliés l'un à l'autre de manière amovible.
